# EUROPEAN PATENT APPLICATION

(11) **EP 1 607 384 A1**
(43) Date of publication of application: **21.12.2005**
(21) Application number: 03774029.7
(22) Date of filing: 17.11.2003
(51) Int. Cl.: C07C 271/20, C07C 271/22, C07C 269/06, A61K 48/00, A61K 31/198, A61P 43/00, C07K 1/08, C07K 5/027, C07K 5/033

(54) **NOVEL FUNCTIONAL PEPTIDE NUCLEIC ACID AND PROCESS FOR PRODUCING THE SAME**

(30) Priority: 19.11.2002 JP 2002335738
(71) Applicant: Credia Japan Co., Ltd., Soraku-gun, Kyoto 619-0273 (JP)
(72) Inventor: IKEDA, Hisafumi, Nagareyama-shi, Chiba 270-0115 (JP); KODAMA, Shuntaro, Katsuse, Fujimi-shi, Saitama 354-0031 (JP); TONOSAKI, Madoka, Kasukabe-shi, Saitama 344-0054 (JP); KITAGAWA, Fumihiko, Noda-shi, Chiba 278-0022 (JP)
(74) Representative: von Füner, Nicolai
(86) International application number: PCT/JP2003/014610
(87) International publication number: WO 2004/046089

(57) **Abstract**

The object of the present invention is to provide functional PNA monomer units and a novel synthesis method for functional PNA oligomers for introducing a plurality of functional molecules at arbitrary locations by means of linkers having various lengths either directly or post-synthetically even in the case of compounds that are unstable in liquid under acidic, alkaline or neutral conditions, along with novel functional PNA monomer units and functional PNA oligomers. This object is achieved by means of a compound in the form of a functional PNA monomer unit, and production method thereof, represented by the following general formula (I): 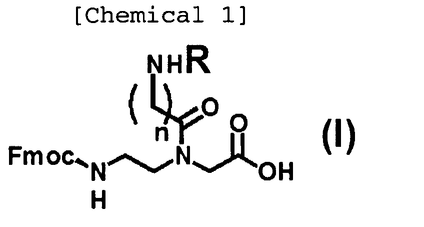 (wherein R represents H, a functional molecule or a protecting group, and n represents an integer of 1 to 11).

## Description

### TECHNICAL FIELD

The present invention relates to novel functional peptide monomers, a production method of the same, and a method for producing functional peptide nucleic acid oligomers using said monomers. More particularly, the present invention relates to precursor PNA monomer units and a production method of the same, and the aforementioned production method in which said monomer units are introduced into a PNA oligomer followed by post-synthetic introduction of one type or two or more types of desired functional molecules.

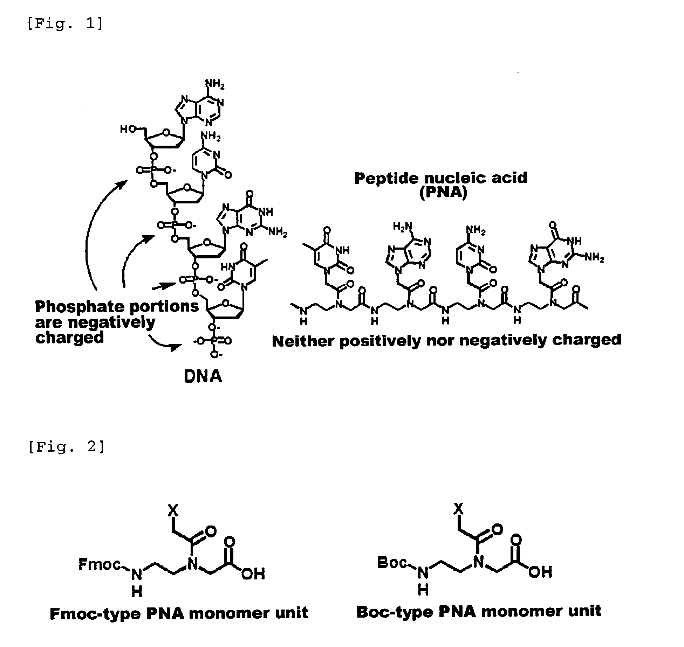

Nucleic acids consist of DNA and RNA that govern the genetic information of living organisms. In contrast, peptide nucleic acids (PNA) refers to modified nucleic acids wherein the sugar-phosphate skeleton of a nucleic acid has been converted to an N-(2-aminoethyl)glycine skeleton (Fig. 1). Although the sugar-phosphate skeletons of DNA/RNA are subjected to a negative charge under neutral conditions resulting in electrostatic repulsion between complementary chains, the backbone structure of PNA does not inherently have a charge. Therefore, there is no electrostatic repulsion. Consequently, PNA has a higher ability to form double strands as compared with conventional nucleic acids, and has a high ability to recognize base sequences. Moreover, since PNA is extremely stable with respect to nucleases and proteases in the living body and is not decomposed by them, studies are being conducted on its application to gene therapy as an antisense molecule.

As a result of using PNA in technology that conventionally used DNA as a medium, it has become possible to compensate for those shortcomings of DNA that were heretofore unable to be overcome. For example, PNA can be applied to "DNA microarray technology" for rapid and large-volume systematic analysis of genetic information, as well as recently developed "molecular beacons" used as probes capable of detecting that a base sequence has been specifically recognized using emission of fluorescent light. Since both of these use DNA lacking enzyme resistance as the medium, strict sampling is required when using these technologies. The satisfying of this requirement is the key to achieving greater sophistication of these technologies.

On the other hand, since PNA is completely resistant to enzymes, by substituting the use of DNA for PNA in DNA microarray technology and molecular beacons, the previously mentioned technical shortcomings can be overcome, leading to expectations of being able to take further advantage of the merits of these technologies.

Although there are many other fields wherein the use of PNA is expected to lead to further advancements in addition to DNA microarray technology and molecular beacons, in these fields it will be necessary to design novel PNA monomers by enabling PNA to function efficiently, namely by realizing the efficient introduction of functional molecules into PNA monomers.

Since ordinary solid-phase peptide synthesis methods are used for PNA oligomer synthesis methods, classification of PNA monomer units according to PNA backbone structure yields the two types consisting of Fmoc type PNA monomer units and tBoc type PNA monomer units (Fig. 2).

Methods for synthesizing Fmoc type PNA monomer units have already been established, and since their oligomer synthesis can be carried out using an ordinary DNA automated synthesizer, synthesis can be carried out on a small scale by the following route: 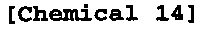 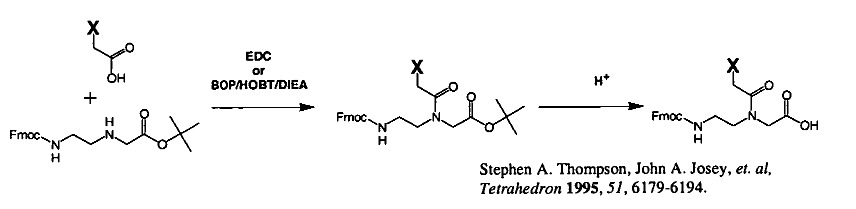 (wherein X represents guanine, thymine, cytosine or adenine).

Initially, tBoc type PNA monomer units like those shown below: 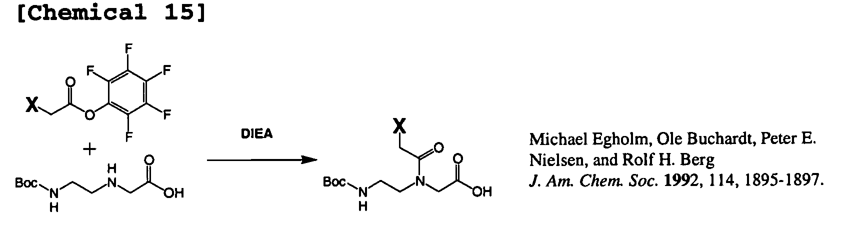 were used and this was followed by the establishment of more efficient synthesis methods.

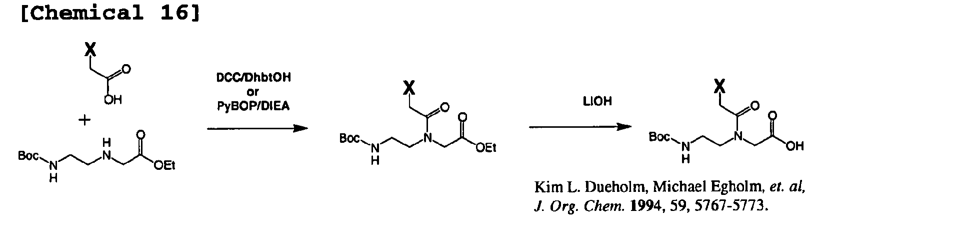

However, since the Fmoc type which can be handled easily was developed as described above, the tBOC type is being used less frequently. Namely, in contrast to using an ultra-strong acid during the elongation reaction and when severing from the resin in the tBoc method, in the Fmoc method, a strong acid in the form of trifluoroacetic acid is used only when severing from the resin, and since the intermediate elongation reaction proceeds under neutral or weakly alkaline conditions, oligomers can be synthesized under extremely mild conditions.

However, when introducing a functional molecule other than the four types of nucleic acid bases of guanine, thymine, cytosine and adenine, such as when introducing a light-emitting molecule, there are many cases wherein the functional molecule to be introduced is unstable under alkaline conditions, and thus a tBoc type of PNA backbone structure that is not used under alkaline conditions is highly useful. A patent application for a "method for producing t-butoxycarbonyl-aminoethylamine and amino acid derivatives" has already been made by the inventors of the present invention as Japanese Patent Application No. 2000-268638.

In addition, there are also five examples of synthesis of monomer units of light-emitting oligo PNA in the prior art. Although all of these use the above route, their yields are either not described or are extremely low (For example, see Patent References 1 and 2 and Non-Patent References 1 to 3). In addition, since the structures of the compounds used have the characteristic of being comparatively stable under alkaline conditions, they are expected to be uable to be produced in good yield using a method similar to the above-mentioned methods of the prior art, namely the following route A, if an unstable chromophore attaches under alkaline conditions.

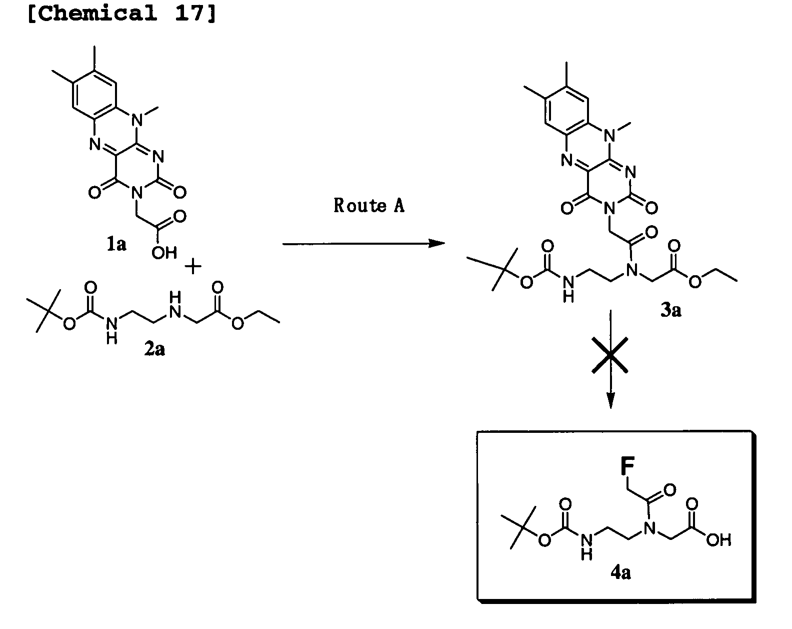

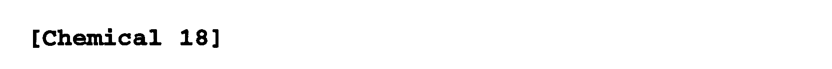

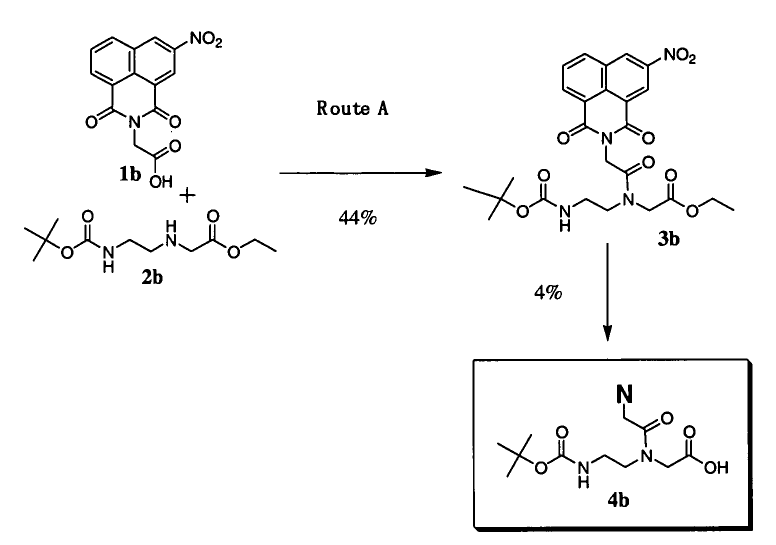

Thus, since there are typically many cases wherein functional molecules such as light-emitting molecules are expensive, methods for synthesizing more pertinent functional PNA, namely methods for extremely rapidly introducing these functional molecules for (1) efficient introduction of functional molecules into a PNA backbone structure in the design of functional PNA monomer units, (2) synthesis routes in consideration of cost performance, and (3) adaptation to applications as gene diagnostic drugs, have been sought.

In consideration of the above problems, the inventors of the present invention found a novel method for producing functional PNA monomers consisting of synthesizing a photofunctional PNA monomer 4 nearly quantitatively by using a t-butoxycarbonylaminoethylamine derivative 6 for the PNA backbone structure, and condensing with an active ester form 5 containing the pentafluorophenyl group of 1 as indicated in the following route B.

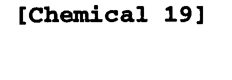

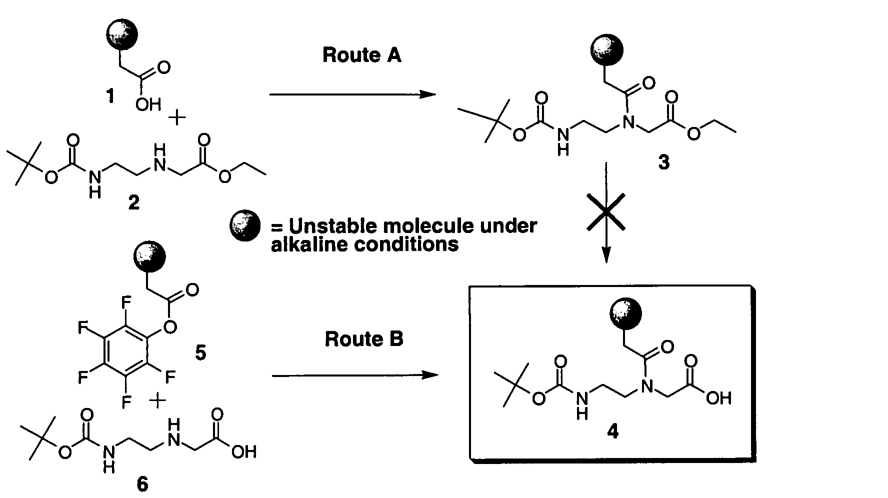

In addition, the inventors of the present invention found a different method for synthesizing functional PNA monomers by using a benzyloxycarbonyl-ω-amino acid derivative instead of the above t-butoxycarbonylaminoethylamine derivative 6 for the PNA backbone structure (route C). Patent applications have already been made for these methods.

Thus, methods for ultimately synthesizing functional PNA are being established industrially that consist of synthesizing functional PNA monomers according to methods using either of the above route B or C, followed by polymerization of those monomers. Namely, it is becoming possible to industrially synthesize large volumes of functional PNA used as PNA probes using existing functional PNA synthesis methods.

On the other hand, improvements are also being made on methods for producing functional PNA for the purpose of improving cost performance and allowing ultra-high-speed introduction of functional molecules. For example, a method has been reported in which functional molecules are introduced into PNA oligomers post-synthetically by using the following precursor PNA monomer unit as a different approach from the method described above using functional PNA monomer units (see Non-Patent Reference 4).

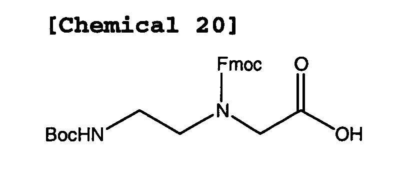

In this method, after introducing the above precursor PNA monomer unit into a PNA oligomer, functional PNA is synthesized by additionally introducing a functional molecule.

However, this method has the disadvantage of there being limitations on the types of functional molecules that can be introduced.

For example, as indicated below, the commercially available light-emitting molecule, succinimide ester, is unable to be introduced. Although it is necessary to first introduce a linker such as Fmoc-Gly in order to introduce this light-emitting molecule, the above compound becomes difficult to use as a result of this.

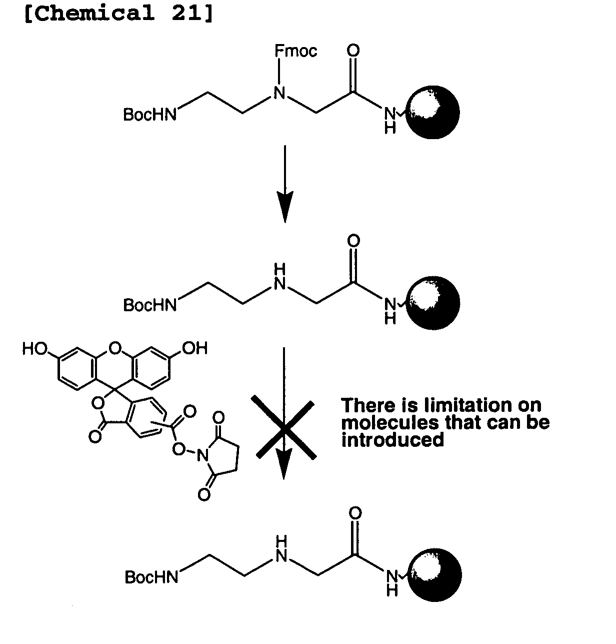

In addition, although DNA oligomers, RNA oligomers and PNA oligomers have been used in the past as fluorescent probes for introducing into cells, in order to introduce these into cells, they must naturally be able to pass through the cell membrane. However, since the surface of the cell membrane has a negative charge, it is extremely difficult to introduce DNA/RNA oligomers that are inherently negatively charged.

On the other hand, although PNA oligomers are electrically neutral, results have been obtained which indicate they are difficult in permeating the cell membrane. Thus, when introducing PNA oligomers into a cell, that introduction must be facilitated by pretreating the membrane surface, or they must be introduced by using a transfection reagent.

However, in the case of introducing PNA oligomers by performing such treatment, even though the probe's function may be demonstrated, there is no guarantee that the behavior inherently demonstrated by the living body will always be accurately represented. Moreover, this is only true in the case of one cell, and in the case of numerous cells (individual body), their use is practically impossible.

On the basis of this current situation and viewpoint, the development of a fluorescent PNA probe having a membrane permeation function is considered to be useful.

It should be noted that fluorescent PNA probes having a membrane permeation function already exist. Examples include (1) a fluorescent PNA probe wherein an oligopeptide having a membrane permeation function is linked to PNA, and (2) a fluorescent PNA probe wherein a phospholipid having a membrane permeation function is linked to PNA. However, the portion of these probes other than the PNA is expected to be decomposed by enzymes such as proteases within cells after they have permeated the cell membrane, thereby causing them to be retained within the cell. Since this leads to excess PNA probes that were unable to capture the target losing their membrane permeation function and having difficulty in moving outside the cell in subsequent washing steps, this means that the gene expression system inherently possessed by the cell cannot be expressed accurately.

In consideration of the aforementioned background, a patent application was previously filed (Japanese Patent Application No. 2002-121667) relating to a synthesis method of a PNA functional oligomer capable of introducing one or more types of functional molecules at an extremely fast rate, and to a compound used in that method.

This method for producing a functional PNA oligomer is characterized by obtaining the desired compound by reacting a PNA monomer unit having thymine or adenine, guanine or cytosine protected by a protecting group, with Fmoc-ω-amino acid-^{Boc}PNA-OH to synthesize a PNA oligomer, followed by post-synthetically introducing a functional group having free carboxylic acid into said PNA oligomer and then removing said protecting group for deprotection.

However, in the aforementioned known production method that uses Fmoc-ω-amino acid-^{Boc}PNA-OH, the functional group also ends up being eliminated during the removal of the protecting group for deprotection in the case where bonding of the functional molecule to the PNA oligomer is weak, thereby preventing the obtaining of adequate yield.

Thus, there continues to be a considerable need for a method capable of more efficiently introducing weakly bonding functional molecules into PNA oligomers in particular.

In addition, it is necessary to use Fmoc-type PNA monomer units that can be used under alkaline conditions to introduce functional molecules for which introduction is difficult in synthesis methods that used functional molecules which are unstable under acidic conditions, namely tBoc-type PNA monomer units. Thus, when also considering that Fmoc-type PNA monomer units are frequently used in automated DNA synthesizers as previously mentioned, there is a considerable demand for novel Fmoc-type PNA monomer units that can be used in automated DNA synthesizers.

Methods for synthesizing Fmoc-type PNA monomer units have been previously reported.

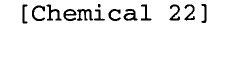

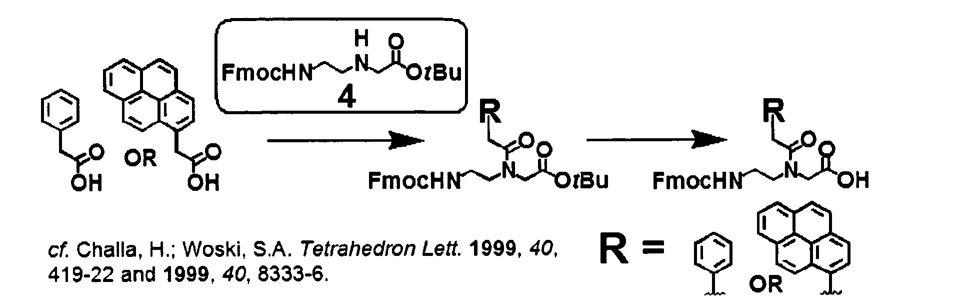

However, the aforementioned method of Woski et al. that uses an Fmoc-type backbone has serious disadvantage. Namely, in the aforementioned method, the distance between the backbone and the functional molecule cannot be adjusted. In addition, since the problem inherent to the Fmoc method of being unable to introduce a functional molecule unless it is stable with respect to strong acid is not overcome, there is also the serious disadvantage of the application range being extremely narrow.

Technical documents relating to the prior art are as indicated below.
[Patent Reference 1]
   Peter E. Nielsen et al.: WO98/5295 A1 pamphlet
[Patent Reference 2]
   Hans-georg Batz et al.: WO98/37232 A2 publication
[Non-patent Reference 1]
   T.A. Tran et al.: "Journal of Peptide Research", 1999, vol. 53, pp. 134 - 145
[Non-patent Reference 2]
   Jesper Lohse et al.: "Bioconjugate Chemistry", 1997, vol. 8, pp. 503 - 509
[Non-patent Reference 3]
   Bruce Armitage et al.: "Nucleic Acid Research", 1998, vol. 26, pp. 715 - 720
[Non-patent Reference 4]
   Oliver Seitz: "Tetrahedron Letters", 1994, vol. 40, pp. 4161 - 4164

Thus, the object of the present invention is to provide functional PNA monomer units and a novel synthesis method for functional PNA oligomers for introducing a plurality of functional molecules at arbitrary locations by means of linkers having various lengths either directly or post-synthetically even in the case of compounds that are unstable in liquid under acidic, alkaline or neutral conditions, along with novel functional PNA monomer units and functional PNA oligomers.

### DISCLOSURE OF THE INVENTION

As a result of extensive research in consideration of the aforementioned problems, the inventors of the present invention surprisingly found that by using a diverse range of novel precursor Fmoc-type PNA monomer units and a production method thereof, the aforementioned problems of the prior art are overcome, and an extremely diverse range of functional PNA monomer units and functional PNA oligomers can be synthesized, thereby leading to completion of the present invention.

Namely, the present invention relates to a compound represented by the following general formula (I): 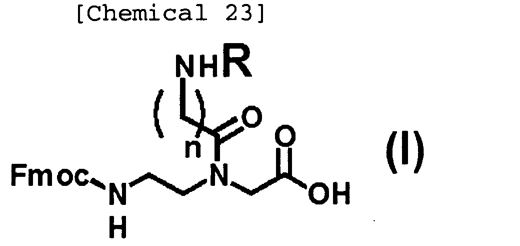 (wherein R represents a hydrogen atom, functional molecule or protecting group, and n represents an integer of 1 to 11).

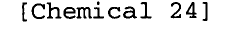

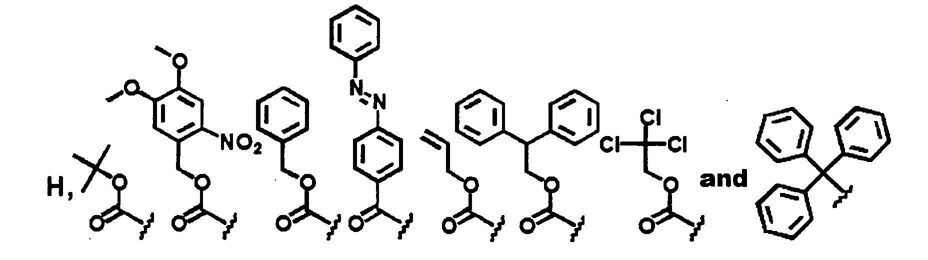

In addition, the present invention relates to a method for producing a compound represented by the following general formula (I): 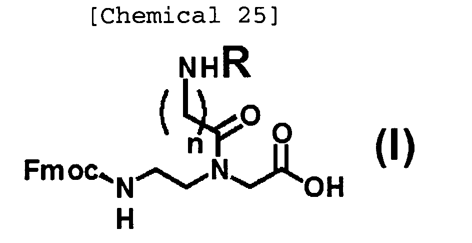 (wherein R represents a functional molecule or protecting group, and n represents an integer of 1 to 11), wherein the aforementioned method contains one of the following steps a) and b):
a)a step of reacting a compound represented by the following general formula (II): 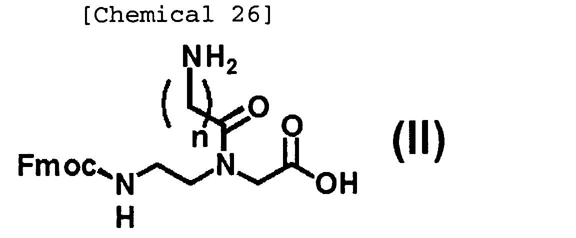 (wherein n represents an integer of 1 to 11) with an active ester derivative with OSu or OPfp of a functional molecule or an isothiocyanate derivative of a functional molecule, and
b)a step of hydrolyzing a compound represented by the following general formula (III): 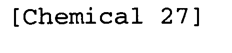 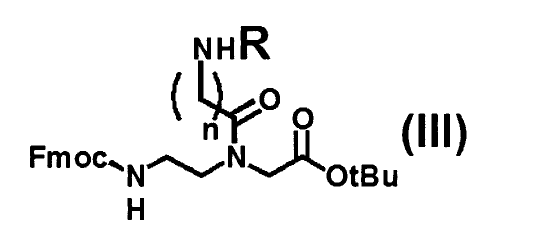 (wherein R represents a functional molecule or a protecting group, and n represents an integer of 1 to 11); as well as,
c)a step of reacting a compound represented by the following general formula (IV): 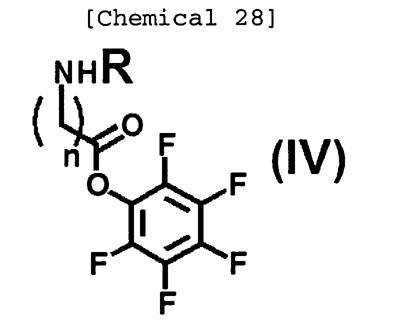 (wherein R represents a functional molecule or a protecting group, and n represents an integer of 1 to 11) with a compound represented by the following general formula (V).

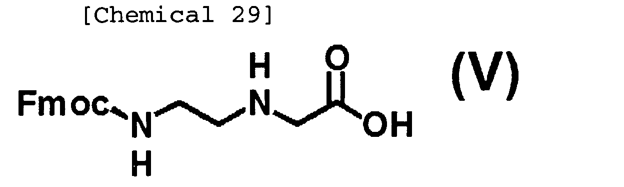

Moreover, the present invention relates to a method for producing a functional PNA oligomer that contains a step of substituting sequentially or simultaneously with a functional molecule H, or a part or all of the protecting groups in B of a PNA oligomer to which is bonded one type or two or more types of a compound represented by the following general formula (I): 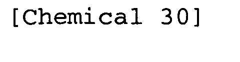 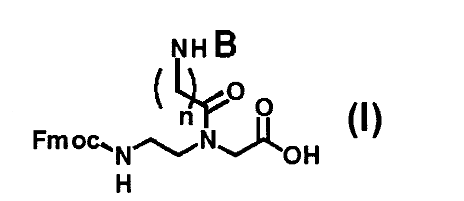 (wherein B represents a hydrogen atom, a functional molecule or a protecting group, and n represents an integer of 1 to 11).

In addition, the present invention relates to the aforementioned method wherein R is selected from the group composed as indicated below.

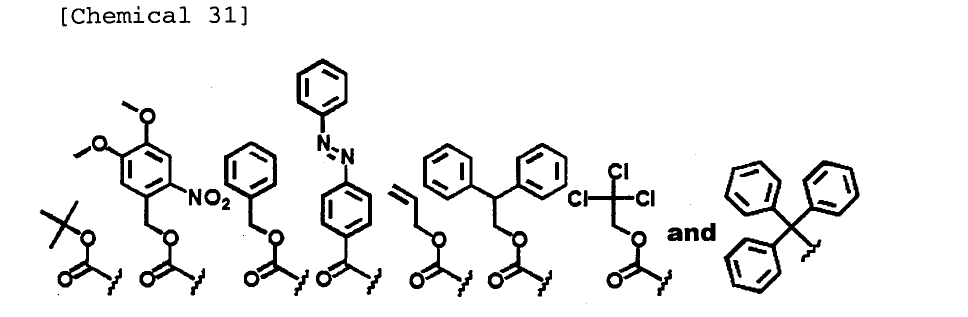

Moreover, the present invention relates to the aforementioned method wherein the compound represented by general formula (I) is produced by one of the steps of the following a) through c):
a)a step of reacting a compound represented by the following general formula (II): 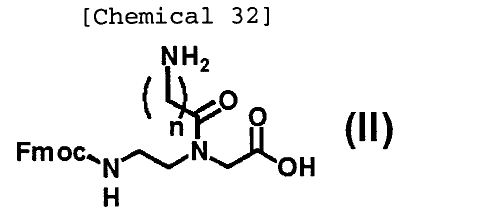 (wherein n represents an integer of 1 to 11) with an active ester derivative with OSu or OPfp of a functional molecule or an isothiocyanate derivative of a functional molecule;
b)a step of hydrolyzing a compound represented by the following general formula (III): 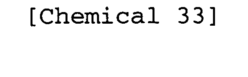 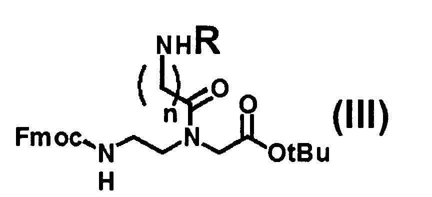 (wherein R represents a functional molecule or a protecting group, and n represents an integer of 1 to 11); and,
c)a step of reacting a compound represented by the following general formula (IV): 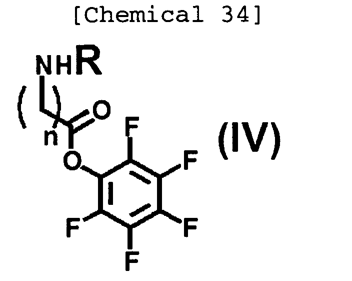 (wherein R represents a functional molecule or a protecting group, and n represents an integer of 1 to 11) with a compound represented by the following general formula (V).

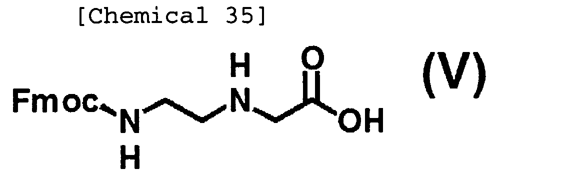

In addition, the present invention relates to the aforementioned method wherein the functional molecule is one type or two or more types selected from a light-emitting molecule, light-dissipating molecule, membrane-permeating functional molecule, organ-selective functional molecule, bactericidal functional molecule, molecule-recognizing functional molecule, photo-crosslinking functional molecule, photosensitizing functional molecule, DNA-bonding molecule and DNA-severing functional molecule.

Moreover, the present invention relates to the aforementioned method wherein the functional molecule contains a light-emitting molecule and a membrane-permeating functional molecule, and the membrane-permeating functional molecule is a water-soluble amino acid.

In addition, the present invention relates to the aforementioned method wherein the functional molecule contains a light-emitting molecule and a light-dissipating molecule, and the light-emitting molecule is FITC, naphthalimide, flavin, FAM, rhodamine, TAMRA, ROX, pyrene or coumarine, and the light-dissipating molecule is Dabcyl, HABA, NDI or Azo.

The present invention succeeds at being able to introduce an Fmoc-type PNA monomer unit, represented by the aforementioned general formula (I) having one type or two or more types of protecting groups or functional molecule groups in a PNA backbone structure, into a PNA oligomer, followed by synthesizing a functional PNA oligomer nearly quantitatively by post-synthetically introducing a functional molecule as a result of substituting sequentially or simultaneously the aforementioned protecting group with a functional molecule.

The aforementioned protecting group is specifically substituted with a functional molecule according to various conditions. Thus, according to the method of the present invention, since suitable protecting groups can be introduced under all possible conditions in order to introduce a functional molecule, an extremely diverse range of functional PNA oligomers can be produced.

In addition, according to a compound according to the present invention, stable oligomer elongation can be carried out under extremely mild alkaline conditions using an Fmoc-type PNA monomer unit represented by the aforementioned general formula (I).

Moreover, an Fmoc derivative having a protecting group of the present invention can be used in numerous automated DNA synthesizers. Thus, according to the present invention, an even wider range of functional PNA oligomers can be produced more rapidly using an automated DNA synthesizer.

As a result of having the aforementioned characteristics, in the production method of the present invention, it is not necessary to use a commercially available succinimide ester for the functional molecule to be introduced, and compounds can be used without problem and introduced quantitatively provided they have a carboxylic acid group. Consequently, the production method according to the present invention has extremely superior cost performance.

In addition, according to the present invention, PNA oligomers can be synthesized in which the same or different functional molecules can be introduced at a plurality of arbitrary locations. Namely, after synthesizing a PNA oligomer using the aforementioned precursor Fmoc-type PNA monomer units, functional groups are post-synthetically introduced either sequentially or simultaneously under conditions preferable for elimination of each protecting group. This is essential for rapidly designing antennapedia that improves the cell membrane permeation function of the PNA oligomer. In addition, according to a production method of the present invention, a molecular beacon that is a PNA oligomer having a photofunctional molecule and light-dissipating molecule can be synthesized easily. A method according to the present invention is extremely superior with respect to this point as well.

Examples of compounds produced in this manner include functional molecules having a cell membrane-permeable molecule derivative and other functional carboxylic acid derivatives. This type of probe can be broadly divided into three regions consisting of a fluorescent labeled region, a cell membrane permeability function region, and a molecule-recognizing region, and can adopt a form in which each is bonded through a linker site.

A commercially available compound or a novel fluorescent labeled PNA monomer unit for which PCT application was previously filed by the inventors of the present invention can be used for the fluorescent labeled compound.

The molecule-recognizing site is synthesized using commercially available PNA units. This compound is characterized by using a novel PNA monomer unit for which patent has already been applied in Japan for the membrane permeability function region. The novel PNA monomer unit is a precursor unit developed to post-synthetically introduce functional molecules, and after having introduced a plurality of these units in a row, the same functional molecules can be introduced as previously described.

Thus, according to the present invention, a diverse range of functional molecules can be introduced easily and extremely efficiently into PNA without being limited to photofunctional molecules and without being subjected to restrictions on linker length.

Examples of such functional molecules include light-emitting molecules such as FITC, naphthalimide, flavin, FAM, rhodamine, TAMRA, ROX, pyrene and coumarine-type, light-dissipating molecules such as Dabcyl, HABA, DNI and Azo-type, membrane-permeable functional molecules such as water-soluble amino acids, organ-selective functional molecules such as lactose and tris-X, bactericidal functional molecules such as tanatin and secropin, molecule-recognizing functional molecules such as biotin and viologen, photo-crosslinking functional molecules such as psoralen, photosensitizing functional molecules such as luciferin, DNA-bonding molecules such as distamycin, Hoechst 33258, and ethidium bromide, and DNA-severing functional molecules such as bleomycin.

Namely, the term "functional" in the present invention refers to all of the various functions newly imparted to compounds by carrying out a specific modification, including one type of two or more types selected from light emission, light dissipation, membrane permeation functionality, organ selectivity functionality, bactericidal functionality, molecule-recognizing functionality, photo-crosslinking functionality, photosensitizing functionality, DNA bonding, and DNA-severing functionality.

Moreover, the term "functional PNA" in the present invention refers not only to the direct bonding of corresponding PNA monomers according to a 2-(N-aminoethyl)glycine skeleton, but also includes a hydrocarbon chain and so forth present therebetween as a linker.

In addition, the term "protecting group" in the present invention refers to all groups in general that protect a specific group under acidic, alkaline or neutral conditions. In the case of group R of a compound of the aforementioned general formula (I) in particular, this refers to a protecting group that protects a side chain amino group, examples of which include linear or cyclic groups having 1 to 20 carbon atoms, and specifically each of the tBoc, Cbz, Nvoc, Fmoc, Alloc, Troc and Trt groups.

### BRIEF DESCRIPTION OF THE DRAWINGS

Fig. 1 is a drawing representing differences in the structure and charge status of DNA and PNA.
Fig. 2 is a drawing representing the structures of two types of PNA monomer units.

### BEST MODE FOR CARRYING OUT THE INVENTION

The following provides a more detailed explanation of the characteristics of the method according to the present invention.

Synthesis of a functional PNA oligomer according to the present invention is typically carried out by:
(1)synthesizing an Fmoc monomer unit;
(2)introducing the Fmoc monomer unit into a PNA oligomer; and,
(3)eliminating the protecting group and introducing a functional molecule.

First, an Fmoc monomer unit of formula (I) is synthesized as necessary according to any of the steps a) through c) shown below.

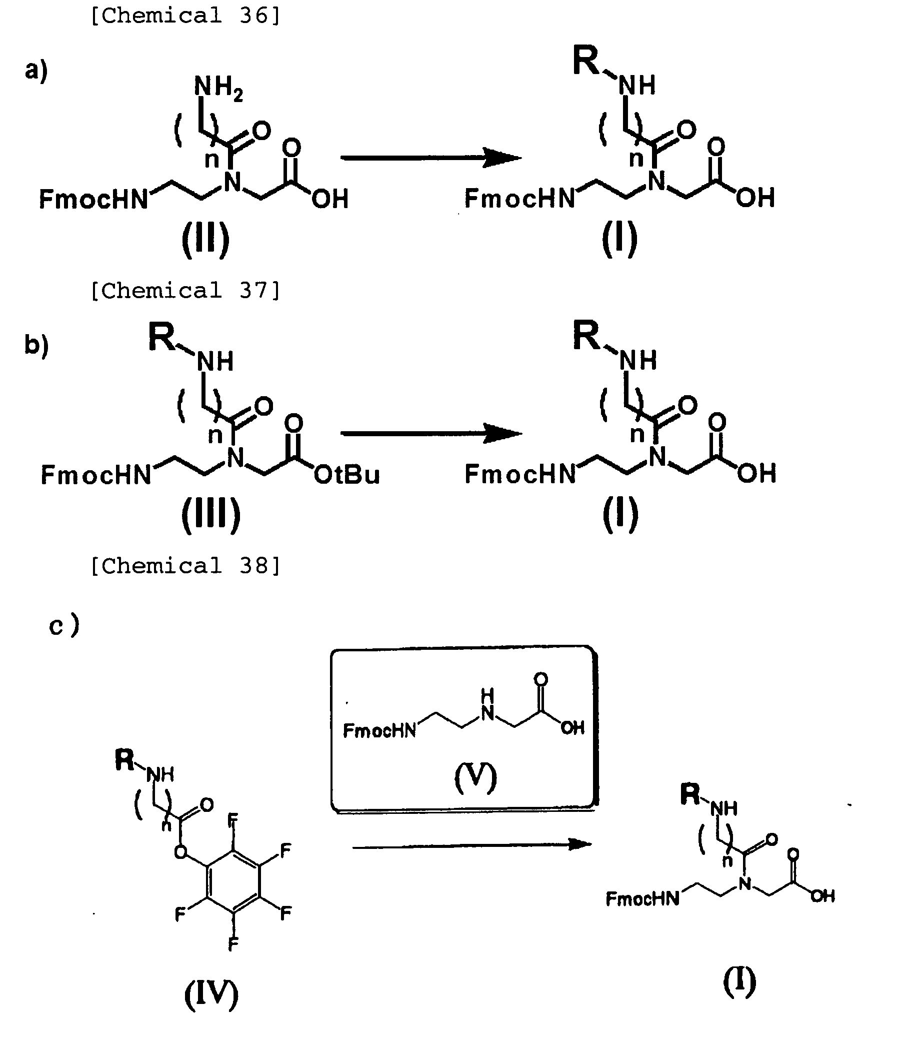

Step a) is a step of synthesizing a PNA monomer unit using an active ester derivative such as OSu or OPfp of a functional molecule or an isothiocyanate derivative such as FITC from a compound represented by formula (II) (ω-amino acid-^{Fmoc}PNA-OH). In this step, a PNA monomer unit can be efficiently synthesized in a single step.

For example, the desired compound of formula (I) can be obtained by condensing an active ester such as a pentafluorophenyl ester or N-hydroxysuccinimidyl ester of a functional molecule at room temperature in the presence of a tertiary amine such as triethylamine.

Although functional molecules are typically expensive, in the case of a method using this step a), since the desired compound can be obtained quantitatively by using only a small amount of functional molecule, the method has extremely superior cost performance.

Furthermore, the compound of the aforementioned formula (II) is preferably synthesized according to the pathway shown below.

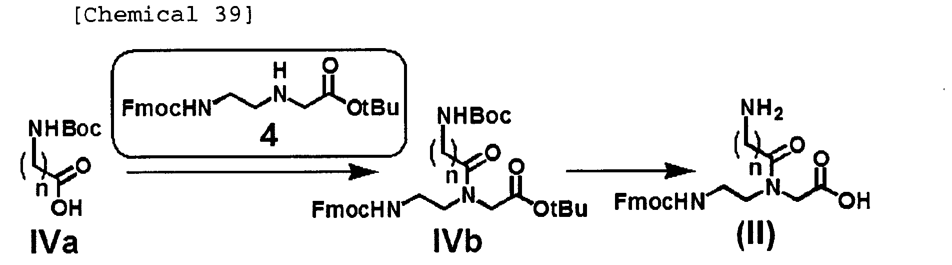

Namely, after first condensing a free carboxylic acid derivative (IVa) of a functional molecule with a key compound 4, the Boc group and tBu group are simultaneously de-protected with strong acid to characteristically obtain a compound of formula (II) in the form of a precursor for synthesizing a functional FmocPNA-OH (compound of formula (I)). Compound (II) allows the desired compound of formula (I) to be rapidly synthesized in a single step by using an active ester of a functional molecule.

More specifically, a condensing agent of DCC, EDCI, HBTU or HATU is added to a DMF (dimethylformamide) solution of Boc-ω-AA-OH (IVa) and FmocPNA-OtBu (4), and allowed to react at room temperature for 12 hours. After filtering the reaction solution and concentrating the filtrate under reduced pressure, the residue is applied to column chromatography to obtain compound IVb.

Compound IVb is then subjected to acid hydrolysis by methylene chloride solution of TFA or a dioxane solution of 4 N HCl to simultaneously de-protect the Boc group and tBu group and obtain the compound of formula (II).

Step b) is a modification of the aforementioned method of Woski et al., and takes into consideration the length of the linker. Namely, although a functional molecule must be present at the optimum steric location for the active site in order to allow the functional molecule to demonstrate its inherent functionality, since a linker is not present in the aforementioned method of Woski et al., the functionality of the functional molecule cannot be maximally demonstrated. On the other hand, in the method of the present invention that uses step b), since the linker length can be changed as desired, it offers the advantage of being able to select a linker corresponding to the properties of the functional molecule, thereby making it possible to demonstrate the function of that functional molecule.

In order to obtain the compound of formula (I) according to step b), for example, the tBu group is de-protected by carrying out acid hydrolysis on the compound of formula (III) with methylene chloride solution of TFA or a dioxane solution of 4 N HCl. Namely, in a method that uses step b), since a free carboxylic acid derivative can be used without converting to an active ester, this method has superior cost performance. Furthermore, since the final reaction process is acid hydrolysis, it is preferable for application to functional molecules that are stable under acidic conditions.

The compound of the aforementioned formula (III) is preferably synthesized according to the pathway shown below.

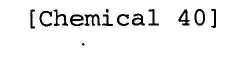

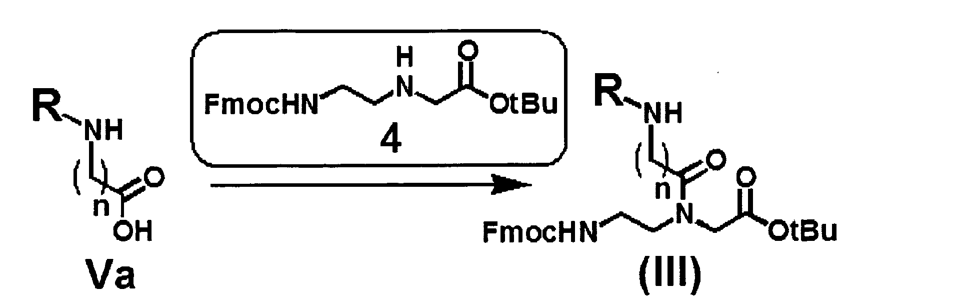

Namely, a free carboxylic acid derivative Va of a ω-amino acid to which a functional molecule has been added is condensed with key compound 4. Subsequently, if a reaction is then carried out that selectively de-protects the tBu group using strong acid, the compound of formula (I) is obtained. Thus, this method offers the advantage of being composed of extremely simple reaction steps, and being able to preferably introduce a functional molecule that is stable in the presence of strong acid.

For example, a condensation agent of DCC, EDCI, HBTU or HATU is added to a DMF (dimethylformamide) solution of the linker-bonded functional molecule R-ω-AA-OH (Va) and FmocPNA-OtBu (4), and allowed to react at room temperature for 12 hours. After filtering the reaction solution and concentrating the filtrate under reduced pressure, the residue is applied to column chromatography to obtain the desired compound of formula (III).

Step c) is a method that uses a compound of formula (V) in which the tBu group of key compound 4 has been de-protected in advance by strong acid to avoid the entering a de-protection process of the tBu group by strong acid that always occurs in the reaction step in case of having used key compound 4 in the manner of the aforementioned step a) or step b). It is necessary to convert the carboxylic acid of the functional molecule to an active ester in order to utilize the compound of formula (V).

Thus, the pentafluorophenyl active ester derivative R-ω-AA-OPfp (compound of formula (IV)) is added to a mixture of an aqueous sodium hydrogencarbonate solution of the compound of formula (V) and acetone, and allowed to react at room temperature for 12 hours. After making the reaction solution acidic with hydrochloric acid (pH 3), aqueous citric acid and ethyl acetate are added followed by a separation procedure. After concentrating the organic phase under reduced pressure, the residue is applied to column chromatography to obtain the desired compound of formula (I).

A method that uses this step c) is characterized in that the Fmoc group in the compound of formula (V) is de-protected in some cases by a tertiary amine required for the action to proceed, and that it needs a step of introducing a linker into the functional molecule. Thus, the method is used preferably in the case of carrying out formation of the desired compound while confirming the formation of intermediates at the laboratory level.

Furthermore, a compound of the aforementioned formula (IV) is preferably synthesized by condensing R-ω-AA-OH (Va) with PfpOH as indicated below.

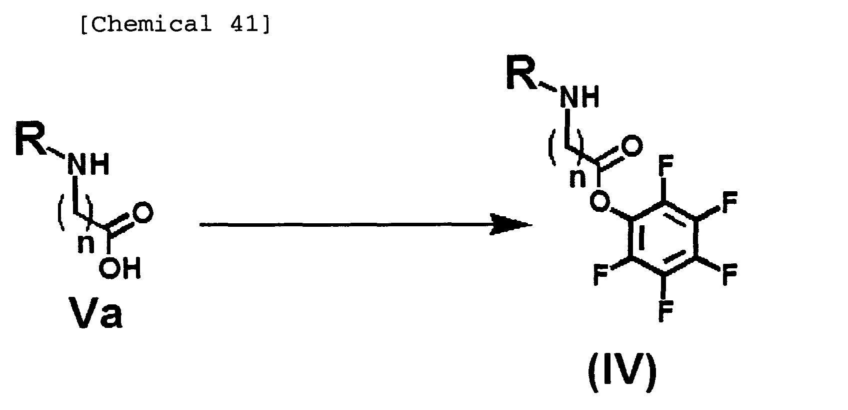

Next, introduction of an Fmoc monomer unit into a PNA oligomer is carried out by the tBoc solid phase method and so forth. Although there are no particular restrictions on the solid-phase support used at this time, MBHA is preferably used.

Fmoc monomers unit represented by formula (I) typically used in the present invention along with their characteristics are as shown below.
(1) ω-AA-^{Fmoc}PNA-OH 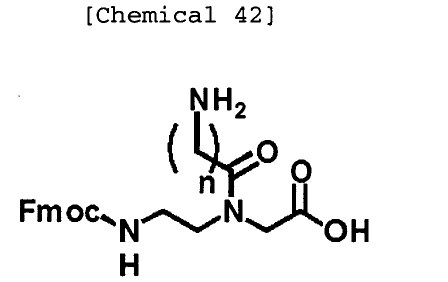
   This is used as a precursor for a functional PNA monomer unit. The symbol "AA" represents an amino acid in the present specification.
(2) Azo-ω-AA-^{Fmoc}PNA-OH 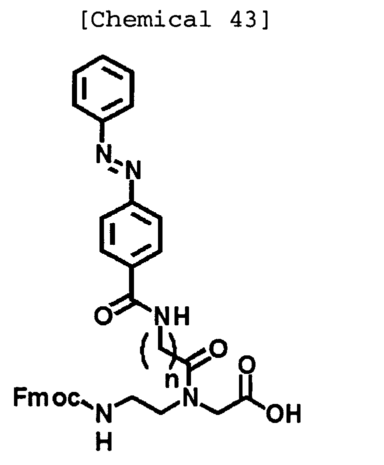
   This example shows that a functional PNA monomer unit can be synthesized quantitatively by reacting with the OSu active ester form of a functional molecule. However, the functional molecule is not limited to Azo.
(3) Trt-ω-AA-^{Fmoc}PNA-OH 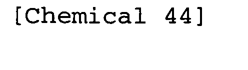 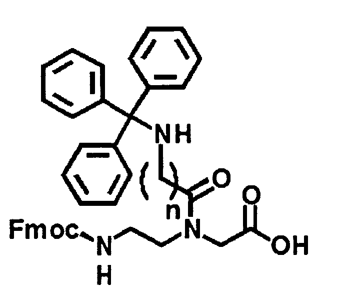
   This is used as a precursor PNA monomer unit during solid-phase synthesis. Since the Trt group can be de-protected with 50% aqueous acetic acid solution, the amino group can be released while the oligomer is bound to the resin. This is characterized by being able to successively introduce functional molecules therein.
(4) tBoc-ω-AA-^{Fmoc}PNA-OH 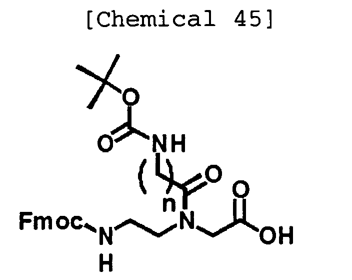
   This is used as a precursor PNA monomer unit during solid-phase synthesis. Although it is stable in the oligomer elongation reaction, since an amino group is released when severing the oligomer from the resin using trifluoroacetic acid (TFA), this can be used while distinguishing between Trt-ω-AA-^{Fmoc}PNA-OH and Cbz-ω-AA-^{Fmoc}PNA-OH.
(5) Nvoc-ω-AA-^{Fmoc}PNA-OH 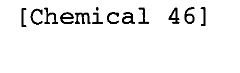 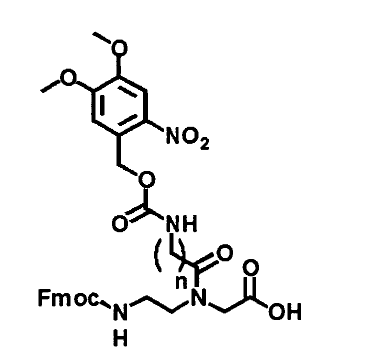
   This is used as a precursor PNA monomer unit during solid-phase synthesis. Since the Nvoc group can be de-protected by irradiating with light, the amino group can be released while the oligomer is bound to the resin. Since this is completely unstable with respect to TFA, the Nvoc group should be de-protected by irradiating with light prior to severing the oligomer from the resin. This is preferably used in elongation reactions.
(6) Fmoc-ω-AA-^{Fmoc}PNA-OH 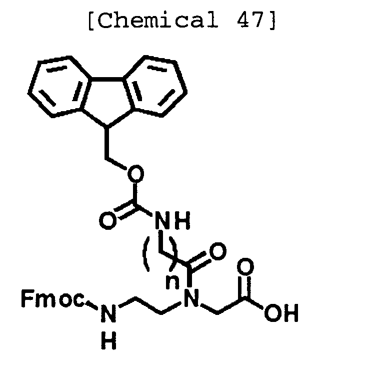
   This is used as a precursor PNA monomer unit during solid-phase synthesis. Since the Fmoc group can be selectively de-protected by piperidine treatment, the amino group can be released while the oligomer is bound to the resin. This is preferably used when producing a dendrimer structure.
(7) Alloc-ω-AA-^{Fmoc}PNA-OH 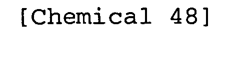 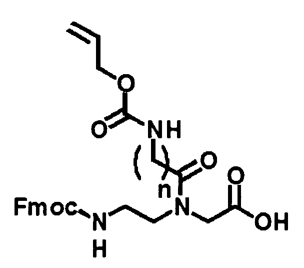
   This is used as a precursor PNA monomer unit during solid-phase synthesis. Since the Alloc group can be selectively de-protected with Pd(PPh3)4, the amino group can be released while the oligomer is bound to the resin.
(8) Cbz-ω-AA-^{Fmoc}PNA-OH 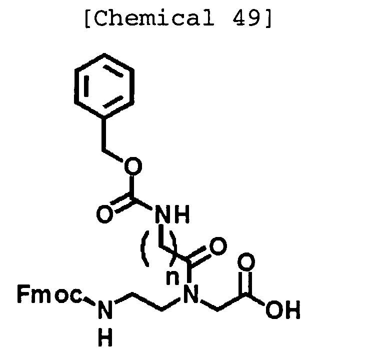
   This is used as a precursor PNA monomer unit during solid-phase synthesis. Although it is stable during the oligomer elongation reaction and when severing the oligomer from the resin using TFA, since the amino group is released by catalytic reduction, it can be used while distinguishing between Trt-ω-AA-^{Fmoc}PNA-OH and tBoc-ω-AA-^{Fmoc}PNA-OH.
(9) Troc-ω-AA-^{Fmoc}PNA-OH

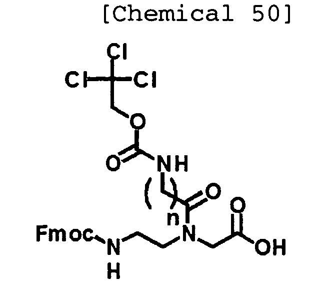

This is used as a precursor PNA monomer unit during solid-phase synthesis. Since the Troc group can be selectively de-protected with 10% aqueous zinc acetate solution, the amino group can be released while the oligomer is bound to the resin. In addition, this is resistant to TFA and catalytic reduction, it is able to remain stable even after severing from the resin, and can be used separately from Cbz-ω-AA-^{Fmoc}PNA-OH.

Finally, substitution is carried out according to the functional molecule of the protecting group. This substitution is carried out sequentially or simultaneously in consideration of the aforementioned characteristics of each Fmoc monomer unit.

Furthermore, although there are no particular limitations on the length of the linker in a compound of formula (I) in the present invention provided that n is within the range of 1 to 11, a linker length of n = 1 to 5 is preferable since it is advantageous in terms of the stability of the linker section when synthesizing a compound of formula (I) and when synthesizing a PNA oligomer.

As has been described above, differing from methods of the prior art that require the synthesis of an active ester form that is used to synthesize functional monomers, functional molecules can be used as such in a method according to the present invention. In addition, since the substitution conditions of functional molecules differ for each protecting group, various functional molecules can be introduced at desired locations after having synthesized a PNA oligomer having a protecting group. Thus, according to a method of the present invention, parallel PNA probes that were difficult to be synthesized in the prior art can be synthesized rapidly and over a diverse range.

A functional PNA oligomer can be synthesized as shown below, for example, by polymerizing a compound of formula (I) according to the present invention: 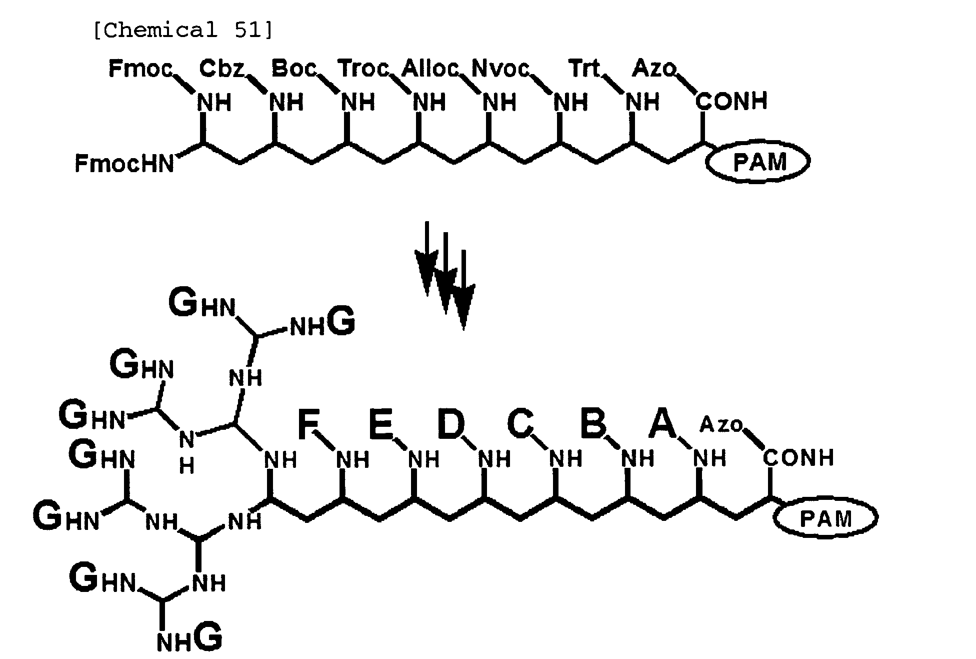 (wherein A through G represent functional carboxylic acid derivatives).

Namely, if a compound of formula (I) according to the present invention is used, all types of functional PNA oligomers can be synthesized that conform to the properties of functional molecules by precisely setting the conditions. The use of a compound of formula (I) is advantageous in terms of being able to efficiently synthesize a dendrimer having a branched structure by using Fmoc-ω-AA-^{Fmoc}PNA-OH in particular.

The following provides an explanation of more concrete examples. The first example is an example in which one functional molecule each is introduced one at a time before and after severing the oligomer from the resin using the aforementioned Trt-ω-AA-^{Fmoc}PNA-OH and tBoc-ω-AA-^{Foc}PNA-OH. In addition, a second example is an example of additionally introducing a functional molecule after severing the oligomer from the resin by using Troc-ω-AA-^{Fmoc}PNA-OH.

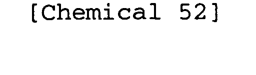

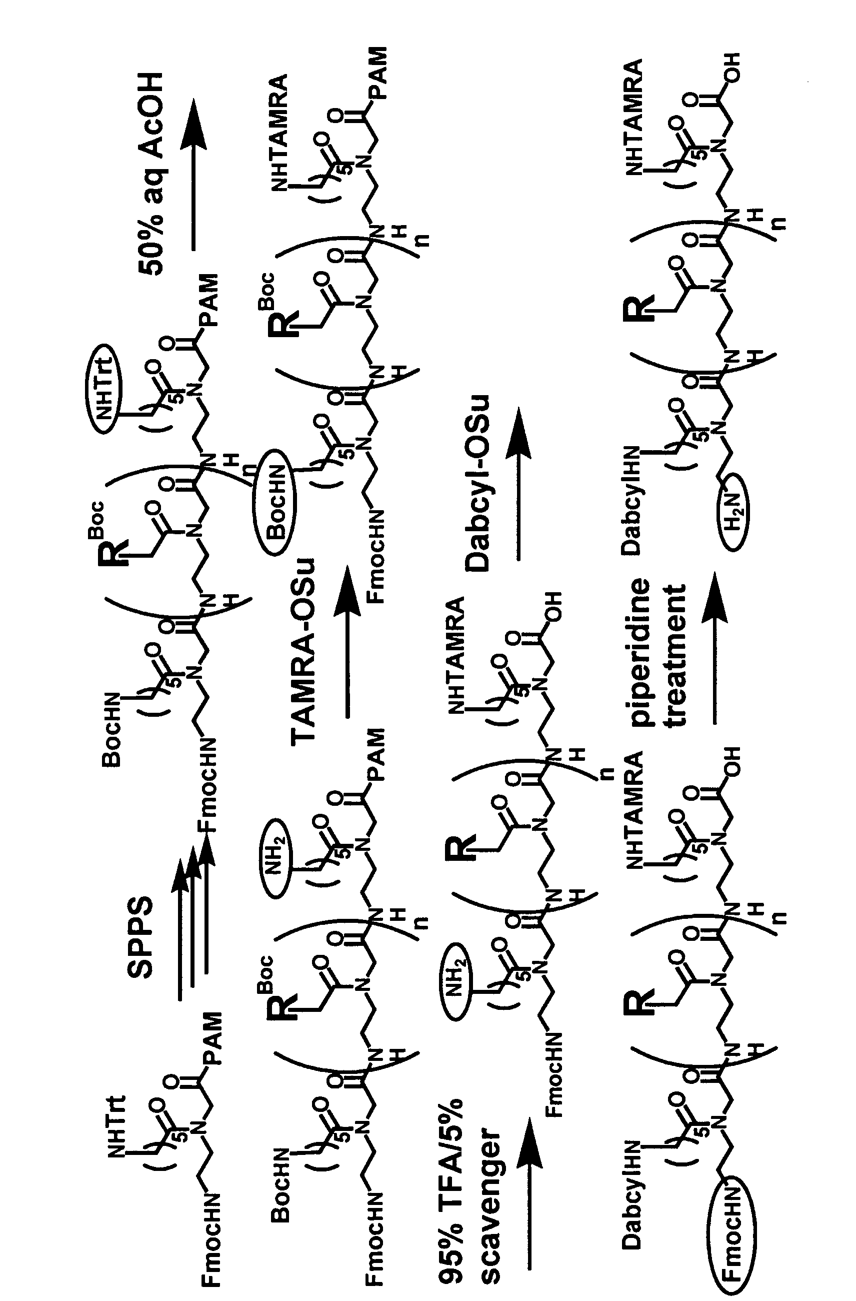

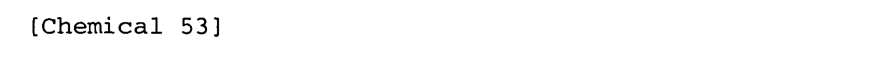

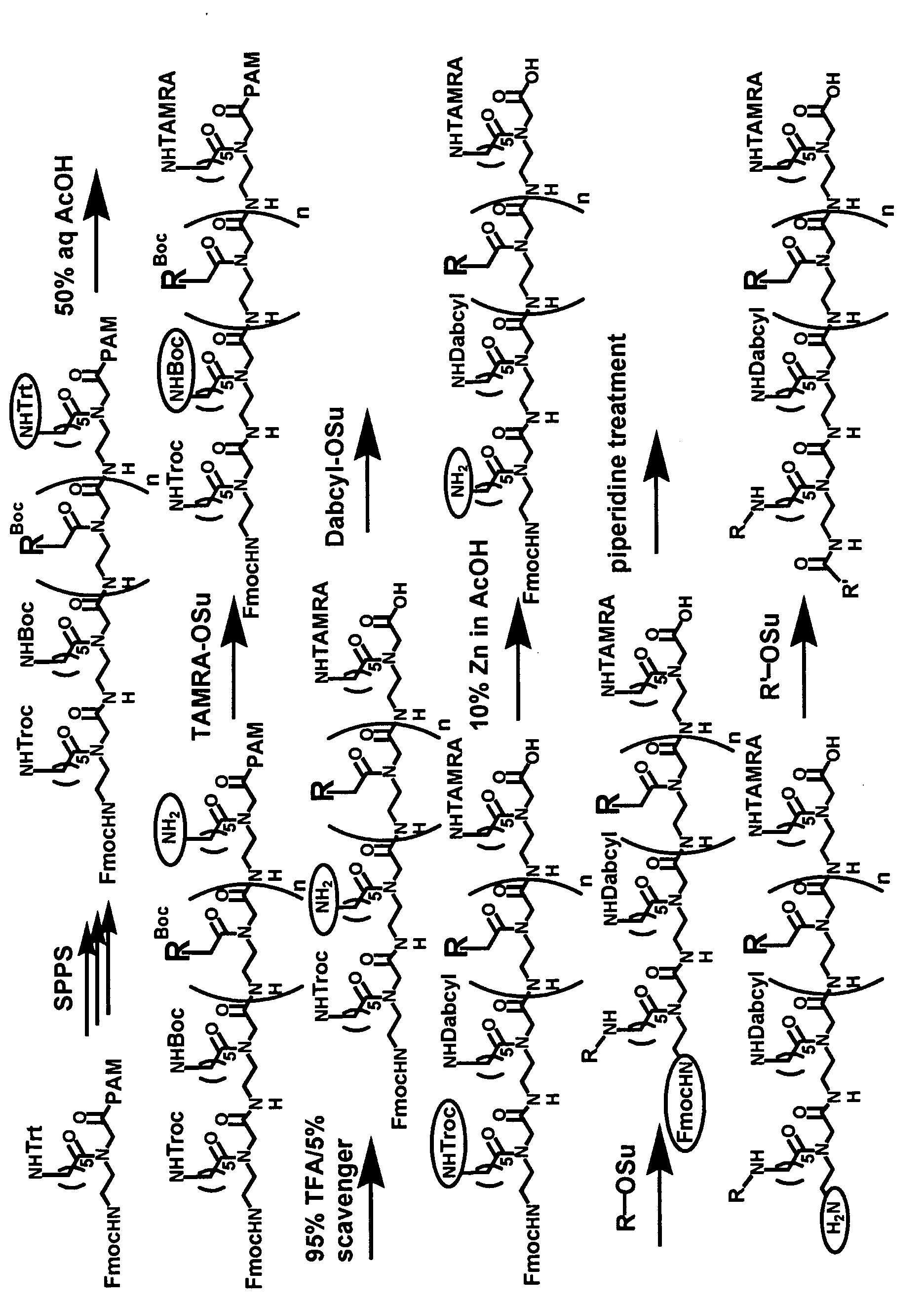

In this manner, if a compound of formula (I) according to the present invention is used, since a plurality of functional molecules can be introduced, a compound is preferably synthesized in which, for example, a functional molecule is a cell membrane-permeable functional molecule derivative. Such a compound typically has functional carboxylic acid derivatives such as a cell membrane-permeable functional molecule derivative and photofunctional molecule, and namely is a compound in which functional molecules are introduced at a plurality of sites including the terminal that is imparted with a plurality of functions as a result of their introduction.

In addition, the functions of the molecules can be made to be more reliable by preventing interference between each of the functional sites and base sequence recognition regions as a result of introducing linker sites. PNA, PNA monomers and PNA oligomers that contain linker sites either at their terminals and/or internally are also included in the terms PNA, PNA monomer and PNA oligomer used in the present specification.

In addition to the aforementioned linker sites contained as sites for preventing interference between these sites or regions, linker length can also be selected as desired.

Although examples of groups that compose linker sites include linear or branched hydrocarbons and their ether forms, linear hydrocarbon groups are preferable in terms of ease of introduction and cost, while linear hydrocarbon groups having 1 to 6 carbon atoms are particularly preferable. In addition, ether forms are also preferable in terms of their universality.

The aforementioned compounds in which a plurality of functional molecules have been introduced are preferably synthesized using, for example the method of Koch, T.; Hansen, H.F.; Andersen, P.; Larsen, T.; Batz, H.G.; Otteson, K.; Orum, H.: J. Peptide Res. 1997, 49, 80-88.

A base sequence recognition site can be converted to an oligomer by solid-phase synthesis using various types of commercially available PNA monomers. Commercially available Boc-7-aminoheptanoic acid or Boc-6-aminocaproic acid and so forth can be used for linker sites.

If a photofunctional molecule is introduced as one functional molecule, the molecule can be fluorescent-labeled and compounds can be synthesized having other functions as well. Although various fluorescent emission wavelengths can be selected using commercially available active ester-type fluorescent labeled compounds such as commercially available FITC, FAM, ROX, TAMRA and pyrene for this type of fluorescent labeling site, the fluorescent-labeled compound that is introduced is not limited to these. In addition, according to a method of the present invention, a light-dissipating molecule such as Dabcyl can also be introduced easily.

A membrane permeability function is an example of another function that can be introduced into a compound of the present invention. Although arginine is an example of a functional molecule that is able to improve membrane permeability, water-soluble amino acids such as lysine and serine can also be used preferably.

These probes are characterized by "having complete enzyme resistance since they are all of the PNA type". Namely, previous probes having a membrane permeability function consisted primarily of those in which the PNA and peptide chain or phospholipid having the membrane permeability function were covalently bonded. Although these known probes have a superior membrane permeability function, once they enter the cell, the peptide chain or phospholipid is expected to be decomposed by enzyme groups. Thus, these have the disadvantage of a decomposed probe that is not recognized as a target being unable to be completely removed in the washing process.

In contrast, a probe capable of being synthesized according to the present invention enables accurate quantitative determination of the amount of expressed gene since probe that does not recognize the target is able to be completely removed in the washing process as a result of not being subjected to enzyme decomposition within the cell.

Furthermore, in addition to compounds having these functions, organ-selective functional molecule such as lactose and tris-X, bactericidal functional molecules such as tanatin and secropin, and molecule-recognizing functional molecules such as viologen can also be introduced without limitation according to the present invention, and such compounds can be provided in large volume for practical use at low cost.

Moreover, a molecular beacon having both a photofunctional molecule and light-dissipating molecule is included in the probes using a production method according to the present invention. A molecular beacon refers to a probe that detects that a base sequence has been specifically recognized using the emission of fluorescent light, and is an important compound in genetic engineering.

Although conventional molecular beacons were unsuitable for storage due to their low level of enzyme resistance, since a probe according to the present invention uses a probe PNA backbone, it has superior stability.

Examples of the aforementioned light-emitting molecule include FITC, naphthalimide, flavin, FAM, rhodamine, TAMRA, ROX, pyrene and coumarine, while examples of the light-dissipating molecule include Dabcyl, HABA, NDI and Azo.

Although the following provides a more detailed explanation of the present invention using its examples, the scope of the present invention is not limited to these examples.

### (Example 1)

### Synthesis of C5-^{Fmoc}PNA-OH

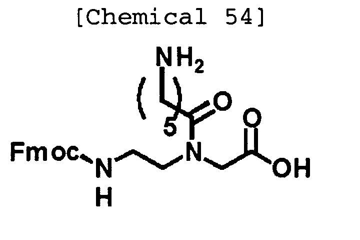

DCC (2.06 g, 10.0 mmol) was added to a dimethylformamide solution (DMF; 10 mL) of Boc-C5-OH (1.15 g, 4.5 mmol) and ^{Fmoc}PNA-OtBu(2.0 g, 5.0 mmol) followed by stirring at 0°C for 2 hours and then at room temperature for 15 hours. After filtering the reaction solution, the filtrate was concentrated under reduced pressure. The residue was applied to silica gel column chromatography (0-4% MeOH/CH₂Cl₂) to obtain 1.16 g (42%) of Boc-C5-^{Fmoc}PNA-OtBu in the form of a white powder. ¹H NMR (CDC13) δ7.75 (brd, J = 7.2 Hz, 2 H), 7.59 (brd, J = 6.4 Hz, 2 H), 7.38 (brt, 2 H), 7.29 (brt, 2 H), 4.36 (ma) and 4.33 (mi) (brd, J = 6.8 Hz, 2 H), 4.19 (t, J = 6.8 Hz, 1 H), 3.94 (mi) and 3.90 (ma) (s, 2 H), 3.53 (mi) and 3.48 (ma) (brs, 2 H), 3.35 (brs, 2 H), 3.08 (ma) and 3.04 (mi) (brs, 4 H), 2.33 (ma) and 2.18 (mi) (brt, J = 7.2 Hz, 2 H), 1.62 (m, 2 H), 1.47 (brs, 9 H), 1.42 (ma) and 1.41 (mi) (s, 9 H), 1.31 (m, 2 H)□

Boc-C5-^{Fmoc}PNA-OtBu (250.0 mg, 0.41 mmol) was dissolved in 4 N HCl in dioxane (2 mL) and stirred at room temperature for 15 hours. This was then concentrated under reduced pressure and the resulting residue was applied to silica gel column chromatography (10-30% MeOH/CH₂Cl₂) to obtain C5-^{Fmoc}PNA-OH (48.0 mg, 26%) in the form of a light pink powder. ¹H NMR (CDCl3)δ 7.71 (brd, J = 6.8 Hz, 2 H), 7.54 (brd, J = 7.2 Hz, 2 H), 7.30 (brt, 2 H), 7.22 (brt, J = 7.2 Hz, 2 H), 4.26 (ma) and 4.23 (mi) (brt, J = 6.8 Hz, 2 H), 4.10 (brt, 1 H), 3.99 (ma) and 3.94 (mi) (s, 2 H), 3.40 (q, J = 6.1 Hz, 2 H), 2.82 (m, 4 H), 2.79 (mi) and 2.75 (ma) (d, J = 8.7 Hz, 2 H), 2.34 (ma) and 2.09 (mi) (brt, J = 7.6 Hz, 2 H), 1.6 - 1.3 (m, 4 H).

### (Example 2)

### Synthesis of tBoc-GABA-^{Fmoc}PNA-OH

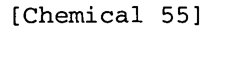

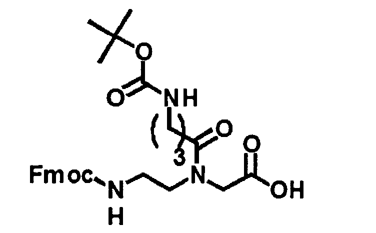

NaHCO₃ (74.1 mg, 0.88 mmol) was added to a mixed solution of acetone (4.8 mL) and water (0.7 mL) followed by dissolving tBoc-GABA-OPfp (200 mg, 0.54 mmol) and ^{Fmoc}PNA-OH (74.1 mg, 0.29 mmol) therein and stirring at room temperature for 15 hours. After adjusting the pH of the solution (0°C) to 3.0 with cold 1 N hydrochloric acid and adding 1% aqueous citric acid solution (15 mL), the mixture was extracted with ethyl acetate (20 mL) and the organic layer was washed with a saturated aqueous NaCl solution (15 mL). The organic layer was dried over anhydrous magnesium sulfate and the solution was concentrated and then purified by silica gel column chromatography (2-7% MeOH/CH₂Cl₂) to obtain tBoc-GABA-^{Fmoc}PNA-OH (71.0 mg, 46%) in the form of a white powder. ¹H-NMR(CDCl₃)δ7.74 (brd, J = 5.3 Hz, 2 H), 7.57 (brd, 2 H), 7.38 (brt, 2 H), 7.29 (brt, J = 5.2 Hz, 2 H), 4.36 (ma) and 4.32 (mi) (brd, J = 6.2 Hz, 2 H), 4.213 (mi) and 4.19 (ma) (m, 1 H), 4.07 (mi) and 4.03 (ma) (s, 2 H), 3.55 (mi) and 3.50 (ma) (brs, 2 H), 3.36 (ma) and 3.26 (mi) (brs, 2 H), 3.08 (m, 2 H), 2.41 (ma) and 2.27 (mi) (brt, 2 H), 1.79 (m, 2 H), 1.41 (m, 9 H); HRMS (FAB⁺) calcd for C₂₈H₃₆N₃O₇ [(M+H)⁺] 526.2553, observed 526.2546.

### (Example 3)

### Synthesis of tBoc-C5-^{Fmoc}PNA-OH

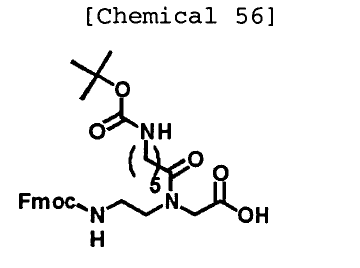

NaHCO₃ (221.8 mg, 2.64 mmol) was added to a mixed solution of acetone (14.3 mL) and water (2.0 mL) followed by dissolving tBoc-C5-OPfp (454.6 mg, 1.14 mmol) and ^{Fmoc}PNA-OH (300.0 mg, 0.88 mmol) therein and stirring at room temperature for 3 days. After adjusting the pH of the solution (0°C) to 3.0 with cold 1 N hydrochloric acid and adding 1% aqueous citric acid solution (45 mL), the mixture was extracted with ethyl acetate (60 mL) and the organic layer was washed with a saturated aqueous NaCl solution (45 mL). The organic layer was dried over anhydrous magnesium sulfate and the solution was concentrated and then purified by silica gel column chromatography (2-7% MeOH/CH₂Cl₂) to obtain tBoc-C5-^{Fmoc}PNA-OH (242.0 mg, 50%) in the form of a white amorphous powder. ¹H-NMR(CDCl₃) δ 7.75 (m, 2 H), 7.57 (m, 2 H), 7.37 (m, 2 H), 7.28 (m, 2 H), 4.34 (ma) and 4.31 (mi) (brd, J = 6.8 Hz, 2 H), 4.22 (mi) and 4.18 (ma) (m, 1 H), 4.06 (mi) and 4.03 (ma) (s, 2 H), 3.56 (mi) and 3.50 (ma) (brs, 2 H), 3.36 (m, 2 H), 3.01 (m, 4 H), 235 (ma) and 2.22 (mi) (brt, 2 H), 1.61 (ma) and 1.60 (mi) (brq, 2 H), 1.41 (m, 9 H), 1.27 (ma) and 1.19 (mi) (brq, 2 H); HRMS (FAB⁺) calcd for C₃₀H₄₀N₃O₇ [(M+H)⁺] 554.2866, observed 554.2867.

### (Example 4)

### Synthesis of Nvoc-GABA-^{Fmoc}PNA-OH

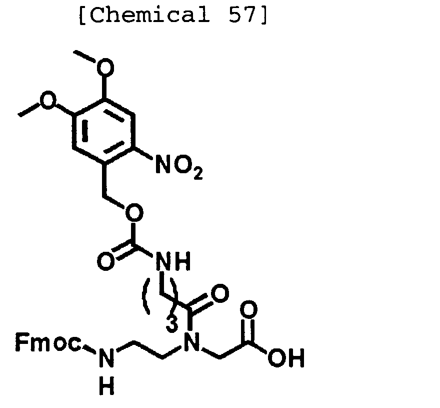

DCC (108.5 mg, 0.53 mmol) was added while cooling with ice to Nvoc-GABA-OH (150.0 mg, 0.44 mmol) and PfpOH (96.8 mg, 0.53 mmol), after which this reaction solution was stirred at 0°C for 30 minutes and then at room temperature for 18 hours. The reaction solution was filtered and the filtrate was concentrated under reduced pressure after which the residue was purified by silica gel column chromatography (2% acetone/CH₂Cl₂) to obtain Nvoc-GABA-OPfp (200.0 mg, 90%) in the form of a yellow powder. ¹H NMR (CDCl₃) δ 7.70 (s, 1 H), 7.00 (s, 1 H), 5.51 (s, 2 H), 5.04 (m, 1 H), 3.97 and 3.95 (each s, 3 H), 3.36 (q, J = 6.4 Hz, 2 H), 2.75 (t, J = 7.1 Hz, 2 H), 2.02 (t, J = 6.9 Hz, 9 H) ; HRMS (FAB⁺) calcd for C₂₀H₁₈N₂O₈ [(M+H)⁺] 509.0983, observed 509.1002.

NaHCO₃ (62.0 mg, 0.74 mmol) was added to a mixed solution of acetone (4.3 mL) and water (0.6 mL) followed by dissolving Nvoc-GABA-OPfp (150.0 mg, 0.30 mmol) and ^{Fmoc}PNA-OH (83.7 mg, 0.25 mmol) therein and stirring at room temperature for 3 days. After adjusting the pH of the solution (0°C) to 3.0 with ice-cooled 1 N hydrochloric acid and adding 1% aqueous citric acid solution (45 mL), the mixture was extracted with ethyl acetate (60 mL) and the organic layer was washed with a saturated aqueous NaCl solution (45 mL). The organic layer was dried over anhydrous magnesium sulfate and the solution was concentrated and then purified by silica gel column chromatography (2-15% MeOH/CH₂Cl₂) to obtain Nvoc-GABA-^{Fmoc}PNA-OH (242.0 mg, 50%) in the form of a yellow powder. ¹H NMR (CDCl₃)δ 8.05 and 7.29 (each s, 1 H), 7.69, 7.54, 7.35, and 7.26 (each brs, 2 H), 5.45 (mi) and 5.41 (ma) (s, 2 H), 4.5 - 4.0 (m, 3 H), 4.0 - 3.7 (m, 8 H), 3.55 (brs, 2 H), 3.37 (ma) and 3.24 (mi) (brs, 2 H), 3.09 (ma) and 3.03 (mi) (brs, 2 H), 2.47 (ma) and 2.33 (mi) (brs, 2 H), 1.86 (ma) and 1.74 (mi) (brs, 2 H); HRMS (FAB+) calcd for C₃₃H₃₇N₄O₁₁ [(M+H)⁺] 665.2459, observed 665.2469.

### (Example 5)

### Synthesis of Nvoc-C5-^{Fmoc}PNA-OH

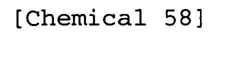

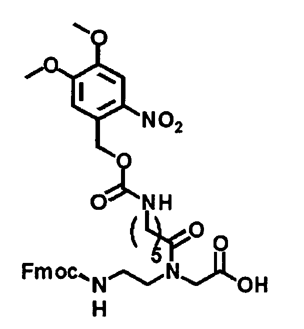

A dioxane solution (1.5 mL) of Nvoc-C1 (24.3 mg, 0.09 mmol) was dropped into an aqueous solution (1.5 mL) of C5-^{Fmoc}PNA-OH (20 mg, 0.04 mmol) and NaHCO₃ (7.4 mg, 0.09 mmol) followed by stirring at room temperature for 1 day. After making the reaction solution acidic (pH 3.0) with hydrochloric acid, water (10 mL) and ethyl acetate (20 mL) were added followed by separation and extraction. The organic layer was dehydrated over anhydrous magnesium sulfate and then concentrated under reduced pressure after which the residue was applied to silica gel column chromatography (2.5-20% MeOH/CH₂Cl₂) to obtain Nvoc-C5-^{Fmoc}PNA-OH (7.2 mg, 24%) in the form of a yellow powder. 1H NMR (CDCl₃) δ 7.69, 7.52, 7.33, and 7.25 (each brs, 2 H), 7.63 and 7.26 (each s, 1 H), 6.92 (brs, 1 H), 5.43 (mi) and 5.36 (ma) (s, 2 H), 4.29 (ma) and 4.17 (mi) (brs, 2 H), 4.12 (mi) and 4.00 (ma) (m, 1 H), 4.04 (mi) and 3.90 (ma) (brs, 6 H), 3.87 (brs, 2 H), 3.55 (mi) and 3.48 (ma) (brs, 2 H), 3.35 (ma) and 3.24 (mi) (brs, 2 H), 3.09 (ma) and 3.02 (mi) (brs, 4 H), 2.36 (ma) and 2.23 (mi) (brs, 2 H), 1.60 (brs, 2 H), 1.47 (mi) and 1.41 (ma) (m, 2 H), 1.31 (ma) and 1.26 (mi) (m, 2 H).

### (Example 6)

### Synthesis of Fmoc-GABA-^{Fmco}PNA-OH

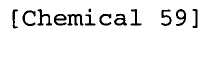

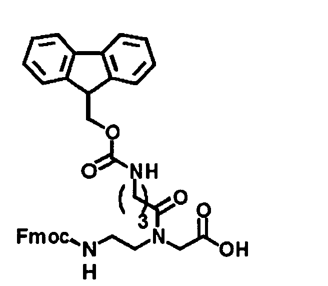

NaHCO₃ (74.1 mg, 0.88 mmol) was added to a mixed solution of acetone (4.8 mL) and water (0.7 mL) followed by dissolving Fmoc-GABA-OPfp (173.5 mg, 0.35 mmol) and ^{Fmoc}PNA-OH (100.0 mg, 0.29 mmol) therein and stirring at room temperature for 3 days. After adjusting the pH of the solution (0°C) to 3.0 with ice-cooled 1 N hydrochloric acid and adding 1% aqueous citric acid solution (45 mL), the mixture was extracted with ethyl acetate (60 mL) and the organic layer was washed with a saturated aqueous NaCl solution (45 mL). The organic layer was dried over anhydrous magnesium sulfate and the solution was concentrated and then purified by silica gel column chromatography (2-15% MeOH/CH₂Cl₂) to obtain Fmoc-GABA-^{Fmoc}PNA-OH (242.0 mg, 50%) in the form of a yellow powder. ¹H NMR (CDCl₃)δ 7.67, 7.49, 7.31, and 7.21 (each m, 4 H), 4.28 (m, 4 H), 4.15 (mi) and 4.10 (ma) (m, 2 H), 3.95 (ma) and 3.75 (mi) (brs, 2 H), 3.46 (mi) and 3.39 (ma) (brs, 2 H), 3.30 (mi) and 3.25 (ma) (brs, 2 H), 3.13 (mi) and 3.08 (ma) (brs, 2 H), 2.35 (ma) and 2.21 (mi) (brs, 2 H), 1.87 (mi) and 1.75 (ma) (brs, 2 H)□

### (Example 7)

### Synthesis of Alloc-ω-AA-^{Fmoc}PNA-OH

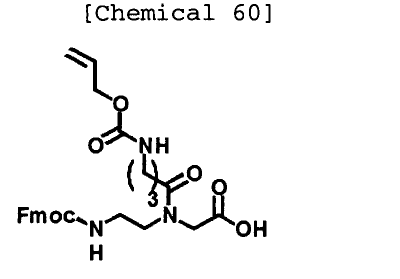

DCC (206.3 mg, 1.0 mmol) was added to an acetone solution (2.5 mL) of Alloc-GABA-OH (243.3 mg, 1.3 mmol) and PfpOH (239.3 mg, 1.3 mmol) followed by stirring the reaction solution at 0°C for 30 minutes and then at room temperature for 15 hours. After filtering the reaction solution, the filtrate was mixed directly with water (1.0 mL) followed by the addition of NaHCO₃ (112.5 mg, 1.3 mmol), dissolving ^{Fmoc}PNA-OH (252.5 mg, 0.67 mmol) therein and stirring at room temperature for 3 days. After adjusting the pH of the solution (0°C) to 3.0 with ice-cooled 1 N hydrochloric acid and adding 1% aqueous citric acid solution (40 mL), the mixture was extracted with ethyl acetate (50 mL) and the organic layer was washed three times with a saturated aqueous NaCl solution (40 mL x 3). The organic layer was dried over anhydrous magnesium sulfate and the solution was concentrated and then purified by silica gel column chromatography (1-15% MeOH/CH₂Cl₂) to obtain Alloc-GABA-^{Fmoc}PNA-OH (200.3 mg, 59%) in the form of a light brown powder. ¹H NMR (CDCl₃) δ 7.69 (brs, 2 H), 7.52 (brs, 2 H), 7.33 (brt, 2 H), 7.24 (brt, 2 H), 5.78 (m, 1 H), 5.1 (m, 2 H), 4.6-4.4 (m, 3 H), 4.29 (ma) and 4.13 (mi) (s, 2 H), 3.94 (m, 2 H), 3.55-3.35 (m, 2 H), 3.26 (m, 2 H), 3.07 (m, 2 H), 2.86 (m, 2 H), 2.35 (ma) and 2.23 (mi) (brs, 2 H), 1.90 (mi) and 1.74 (ma) (brs, 2 H).

### (Example 8)

### Synthesis of Cbz-GABA-^{Fmoc}PNA-OH

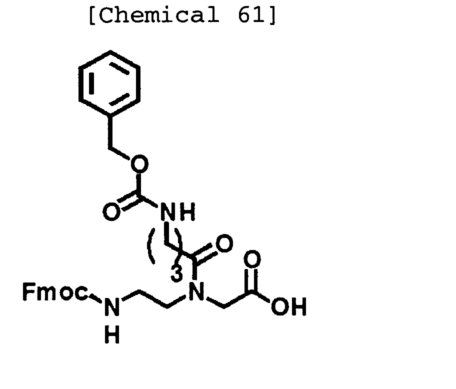

NaHCO₃ (74.1 mg, 0.88 mmol) was added to a mixed solution of acetone (4.8 mL) and water (0.7 mL) followed by dissolving Cbz-GABA-OPfp (177.9 mg, 0.44 mmol) and ^{Fmoc}PNA-OH (100.0 mg, 0.29 mmol) therein and stirring at room temperature for 3 days. After adjusting the pH of the solution (0°C) to 3.0 with ice-cooled 1 N hydrochloric acid and adding 1% aqueous citric acid solution (45 mL), the mixture was extracted with ethyl acetate (60 mL) and the organic layer was washed with a saturated aqueous NaCl solution (45 mL). The organic layer was dried over anhydrous magnesium sulfate and the solution was concentrated and then purified by silica gel column chromatography (2-15% MeOH/CH₂Cl₂) to obtain Cbz-GABA-^{Fmoc}PNA-OH (81.0 mg, 50%) in the form of a white powder. ¹H NMR (CDCl₃) δ 7.73 (brs, 2 H), 7.55 (brs, 2 H), 7.35 (brt, 2 H), 7.28 (brt, 2 H), 7.26 (s, 5 H), 5.03 (mi) and 5.00 (ma) (s, 2 H), 4.4 - 4.1 (m, 3 H), 3.96 (m, 2 H), 3.41 (ma) and 3.31 (mi) (brs, 2 H), 3.27 (ma) and 3.16 (mi) (brs, 2 H), 3.11 (ma) and 3.05 (mi) (brs, 2 H), 2.36 (ma) and 2.23 (mi) (brs, 2 H), 1.86 (mi) and 1.78 (ma) (brs, 9 H).

### (Example 9)

### Synthesis of Troc-GABA-^{Fmoc}PNA-OH

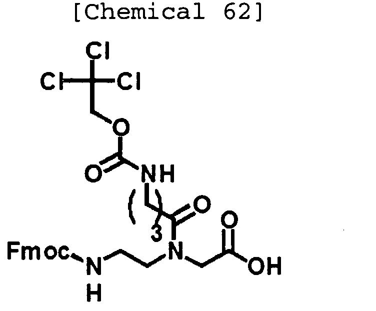

DCC (108.5 mg, 0.53 mmol) was added while cooling with ice to a DMF solution (5 mL) of Troc-GABA-OH (150.0 mg, 0.44 mmol) and PfpOH (96.8 mg, 0.53 mmol), after which this reaction solution was stirred at 0°C for 30 minutes and then at room temperature for 18 hours. The reaction solution was filtered and the filtrate was concentrated under reduced pressure after which the residue was purified by silica gel column chromatography (2% acetone/CH₂Cl₂) to obtain Troc-GABA-OPfp (200.0 mg, 90%) in the form of a white powder. ¹H NMR (CDCl₃) δ 7.70, 7.52, 7.34, and 7.24 (each m, 2 H), 4.64 (mi) and 4.61, (ma) (brs, 2 H), 4.31 (m, 2 H), 4.13 (m, 1 H), 4.2-3.9 (m, 2 H), 3.45 (ma) and 3.42 (mi) (brs, 2 H), 3.27 (ma) and 3.16 (mi) (brs, 2 H), 3.11 (mi) and 3.06 (ma) (brs, 2 H) , 2.38 (ma) and 2.25 (mi) (brs, 2 H), 1.77 (mi) and 1.68 (ma) (brs, 2 H)□

NaHCO₃ (62.0 mg, 0.74 mmol) was added to a mixed solution of acetone (4.0 mL) and water (0.5 mL) followed by dissolving Troc-GABA-OPfp (133.4 mg, 0.30 mmol) and ^{Fmoc}PNA-OH (56.5 mg, 0.25 mmol) therein and stirring at room temperature for 3 days. After adjusting the pH of the solution (0°C) to 3.0 with ice-cooled 1 N hydrochloric acid and adding 1% aqueous citric acid solution (45 mL), the mixture was extracted with ethyl acetate (60 mL) and the organic layer was washed with a saturated aqueous NaCl solution (45 mL). The organic layer was dried over anhydrous magnesium sulfate and the solution was concentrated and then purified by silica gel column chromatography (2-15% MeOH/CH₂Cl₂) to obtain Troc-GABA-^{Fmoc}PNA-OH (46.1 mg, 31%) in the form of a yellow powder. ¹H NMR (CDCl₃)δ 7.70, 7.52, 7.34, and 7.24 (each m, 2 H), 4.64 (mi) and 4.61 (ma) (brs, 2 H), 4.31 (m, 2 H), 4.13 (m, 1 H), 4.2-3.9 (m, 2 H), 3.45 (ma) and 3.42 (mi) (brs, 2 H), 3.27 (ma) and 3.16 (mi) (brs, 2 H), 3.11 (mi) and 3.06 (ma) (brs, 2 H), 2.38 (ma) and 2.25 (mi) (brs, 2 H), 1.77 (mi) and 1.68 (ma) (brs, 2 H) .

### INDUSTRIAL APPLICABILITY

Functional PNA monomer units and a novel synthesis method for functional PNA oligomers for introducing a plurality of functional molecules at arbitrary locations by means of linkers having various lengths either directly or post-synthetically even in the case of compounds that are unstable in liquid under acidic, alkaline or neutral conditions, along with novel functional PNA monomer units and functional PNA oligomers, are provided by the present invention. Thus, since various PNA can be constructed, the present invention is able to make an extremely significant contribution to the chemical industry and fields such as gene therapy.

## Claims

**1.** A compound represented by the following general formula (I): 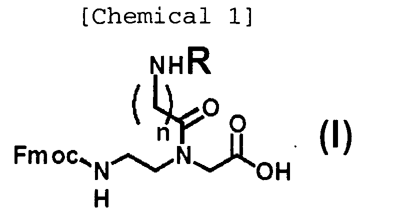 (wherein R is H, a functional molecule, or a protecting group and n represents an integer of 1 to 11).

**2.** The compound according to claim 1, wherein the functional molecule is one type or two or more types selected from a light-emitting molecule, light-dissipating molecule, membrane-permeating functional molecule, organ-selective functional molecule, bactericidal functional molecule, molecule-recognizing functional molecule, photo-crosslinking functional molecule, photosensitizing functional molecule, DNA-bonding molecule and DNA-severing functional molecule.

**3.** The compound according to claim 2, wherein the functional molecule contains a light-emitting molecule and a membrane-permeating functional molecule, and the membrane-permeating functional molecule is a water-soluble amino acid.

**4.** The compound according to claim 2, wherein the functional molecule contains a light-emitting molecule and a light-dissipating molecule, and the light-emitting molecule is FITC, naphthalimide, flavin, FAM, rhodamine, TAMRA, ROX, pyrene or coumarine, and the light-dissipating molecule is Dabcyl, HABA, NDI or Azo.

**5.** The compound according to claim 1, wherein R is selected from the group consisting of

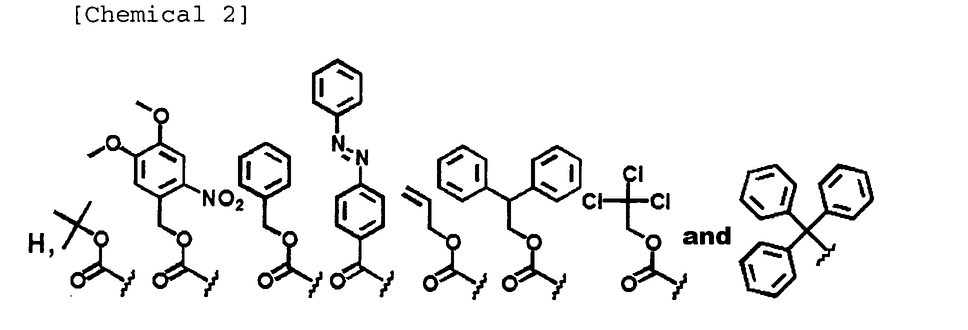

**6.** A method for producing a compound represented by the following general formula (I): 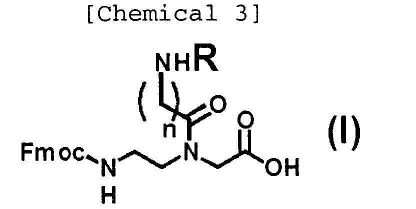 (wherein R represents H, a functional molecule or a protecting group, and n represents an integer of 1 to wherein the aforementioned method contains one of the following steps a) through c):
a) a step of reacting a compound represented by the following general formula (II): 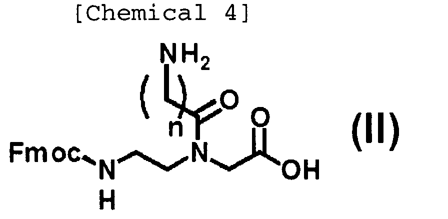 (wherein n represents an integer of 1 to 11) with an active ester derivative with OSu or OPfp of a functional molecule or an isothiocyanate derivative of a functional molecule;
b) a step of hydrolyzing a compound represented by the following general formula (III): 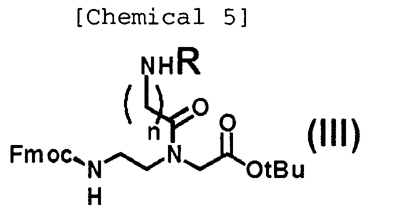 (wherein R represents H, a functional molecule or a protecting group, and n represents an integer of 1 to 11); and,
c) a step of reacting a compound represented by the following general formula (IV): 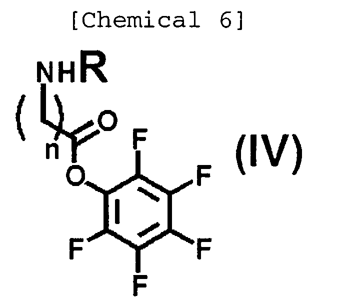 (wherein R represents H, a functional molecule or a protecting group, and n represents an integer of 1 to 11) with a compound represented by the following general formula (V).

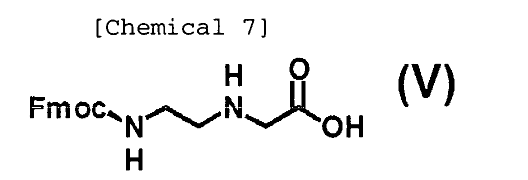

**7.** A method for producing a functional PNA oligomer comprising a step of substituting group R of a PNA oligomer to which is bonded one type or two or more types of a compound represented by the following general formula (I): 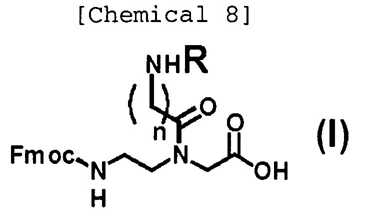 (wherein R represents H, a functional molecule or a protecting group, and n represents an integer of 1 to 11) sequentially or simultaneously with a functional molecule.

**4.** The method according to claim 10, wherein R is selected from the group consisting of the following.

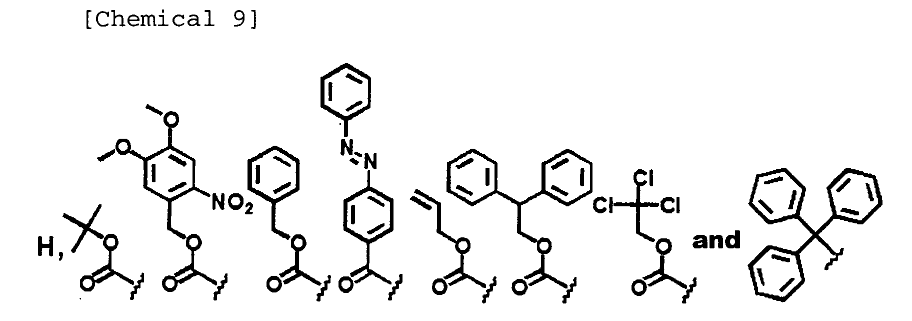

**9.** The method according to claim 6 or 7, wherein the compound represented by general formula (I) is produced by one of the steps of the following a) through c):
a) a step of reacting a compound represented by the following general formula (II): 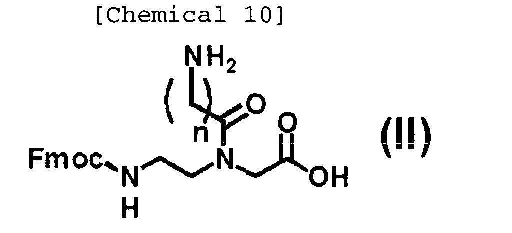 (wherein n represents an integer of 1 to 11) with an active ester derivative with OSu or OPfp of a functional molecule or an isothiocyanate derivative of a functional molecule;
b) a step of hydrolyzing a compound represented by the following general formula (III): 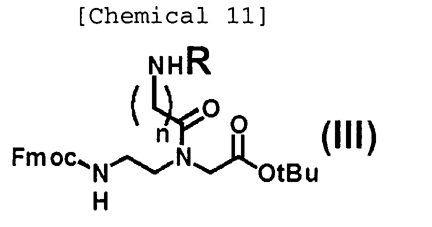 (wherein R represents H, a functional molecule or a protecting group, and n represents an integer of 1 to 11); and,
c) a step of reacting a compound represented by the following general formula (IV): 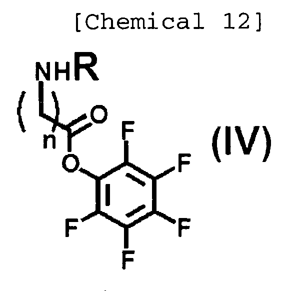 (wherein R represents H, a functional molecule or a protecting group, and n represents an integer of 1 to 11) with a compound represented by the following general formula (V).

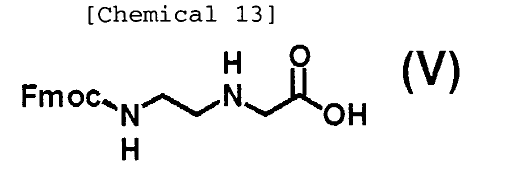

**10.** The method according to any of claims 10 to 12,
wherein the functional molecule is one type or two or more types selected from a light-emitting molecule, light-dissipating molecule, membrane-permeating functional molecule, organ-selective functional molecule, bactericidal functional molecule, molecule-recognizing functional molecule, photo-crosslinking functional molecule, photosensitizing functional molecule, DNA-bonding molecule and DNA-severing functional molecule.

**11.** The method according to claim 10, wherein the functional molecule contains a light-emitting molecule and a membrane-permeating functional molecule, and the membrane-permeating functional molecule is a water-soluble amino acid.

**12.** The method according to claim 10, wherein the functional molecule contains a light-emitting molecule and a light-dissipating molecule, and the light-emitting molecule is FITC, naphthalimide, flavin, FAM, rhodamine, TAMRA, ROX, pyrene or coumarine, and the light-dissipating molecule is Dabcyl, HABA, NDI or Azo.
